# EUROPEAN PATENT APPLICATION

(11) **EP 3 290 033 A2**
(43) Date of publication of application: **07.03.2018**
(21) Application number: 16786772.0
(22) Date of filing: 28.04.2016
(51) Int. Cl.: A61K 31/439, A61K 31/473, A61K 31/47

(54) **PHARMACEUTICAL PACKAGE**

(30) Priority: 29.04.2015 KR 20150060163
(71) Applicant: Samyang Biopharmaceuticals Corporation, Seoul 03129 (KR)
(72) Inventor: YOON, Hye-Jeong, Daejeon 35314 (KR); LEE, Sang-Jun, Daejeon 35216 (KR); LEE, Dong-Ho, Daejeon 34190 (KR)
(74) Representative: Krauns, Christian
(86) International application number: PCT/KR2016/004475
(87) International publication number: WO 2016/175586

(57) **Abstract**

Provided are a pharmaceutical package of palonosetron, comprising a pharmaceutically acceptable container, of which the elution amount of zinc (Zn) is maintained at 50 ppb (parts per billion) or less, and a method of increasing storage stability.

## Description

### [Technical Field]

The present disclosure relates to a palonosetron-including pharmaceutical package and a method of improving the storage-stability of palonosetron.

### [Background Art]

Administration of cancer chemotherapeutic agents including cisplatin induces nausea and vomiting in nearly all cases. Vomiting begins to occur 1 to 2 hours after administration of cisplatin, which is acute vomiting, and vomiting begins to subside by 18 to 24 hours. Then, vomiting begins to develop and peaks 48 to 71 hours, which is delayed vomiting.

Further, nausea and vomiting are the most common complications occurring after anesthesia and surgery. Postoperative vomiting may cause severe complications such as dehydration, electrolyte imbalance, gastric herniation, wound disruption, esophageal tears, muscular fatigue, etc., and it may increase anxiety about additional surgery in patients. In general, postoperative vomiting occurs within 24 hours after surgery in 25 to 40% of surgical patients.

Meanwhile, vomiting may be treated by antagonistic action of 5-HT3 (5-hydroxytryptamine) receptor antagonist at the cerebral functions related to 5-HT3 receptors.

The first-generation 5-HT3 receptor antagonist, ondansetron or granisetron is very effective for acute vomiting, but less effective in preventing delayed vomiting. Therefore, ondansetron or granisetron must be given intravenously once or more times before chemotherapy or radiotherapy is initiated. Thereafter, 5-HT3 receptor antagonist must be given orally in a tablet or elixir form in order to prevent delayed vomiting. Because some anticancer chemotherapeutic agents can induce vomiting for a predetermined period of time or longer even when they are administered only once, a 5-HT3 antagonist must be administered every day until the risk of vomiting has substantially subsided.

US Patent No. 5,202,333 discloses an intravenous formulation of tricyclic 5-HT3 receptor antagonists including a bridged bicyclic amine substituent, such as palonosetron. This document discloses that an administration dose of the tricyclic 5-HT3 receptor antagonist generally ranges from 1 ng to 1 mg, preferably 10 to 100,000 ng per 1 kg of body weight. This document discloses a composition consisting of palonosetron hydrochloride, dextrose monohydrate, citric acid monohydrate, sodium hydroxide, and water for injection, but there is a problem that pharmaceutically acceptable storage stability was not secured.

Palonosetron hydrochloride is marketed under the brand name of ALOXI®. This product is a liquid formulation for single intravenous administration, available as a 5 mL single use vial or a 1.5 mL single use vial. Each 5 mL vial contains 0.25 mg of palonosetron, 207.5 mg of mannitol, citrate buffer, and disodium edetate. Each 1.5 mL vial contains 0.075 mg of palonosetron, 83 mg of mannitol, citrate buffer, and disodium edetate.

Korean Patent Nos. 10-1113084 and 10-1113087 disclose a pharmaceutically stable composition at pH of 4 to 6, including palonosetron, mannitol, citrate buffer, and 0.005 to 1.0 mg/ml of EDTA (ethylenediaminetetraacetic acid) as a chelating agent. In these documents, to secure stability of the pharmaceutically composition including palonosetron, disodium edetate or EDTA is used as a chelating agent. EDTA is used to treat acute and chronic lead poisoning by removing toxic elements including heavy metals such as lead, cadmium, and mercury from the blood stream (chelation therapy). EDTA chelation therapy is approved by the U.S. FDA for use in treating lead and heavy metal poisoning, and also used for emergency treatment of hypercalcemia and control of ventricular arrhythmias associated with digitalis toxicity. However, the most common side effect of EDTA is a burning sensation at the site of the injection, and some people may have an allergic reaction to EDTA. Other serious side effects that have been reported include low blood sugar, diminished calcium levels, headache, nausea, low blood pressure, kidney failure, organ damage, irregular heartbeat, seizures, or even death.

For pharmaceutically effective application of compositions including palonosetron, stability of palonosetron must be improved to secure a pharmaceutically available period. Therefore, a demand for a palonosetron formulation with pharmaceutically effective storage stability still remains.

### [DISCLOSURE]

### [Technical Problem]

Accordingly, an object of the present invention is to provide a pharmaceutical package having improved palonosetron stability.

Another object of the present invention is to provide a pharmaceutical package without a chelating agent such as disodium edentate, etc., and having improved palonosetron stability.

Still another object of the present invention is to provide a method of improving the storage-stability of palonosetron by using the pharmaceutical package.

### [Technical Solution]

An aspect of the present invention provides a pharmaceutical package of palonosetron, including a pharmaceutical composition comprising palonosetron or a pharmaceutically acceptable salt thereof, and a pharmaceutically acceptable container maintaining zinc (Zn) elution amount at 50 ppb (parts per billion) or lower. The pharmaceutically acceptable container can maintain calcium (Ca) elution amount at 30 ppb or lower.

The composition may further comprise a pharmaceutically acceptable aqueous carrier.

The composition may comprise palonosetron or a pharmaceutically acceptable salt thereof at a concentration of 0.01 to 0.5 mg/ml as palonosetron.

The composition may further comprise a buffer, an isotonic agent or a mixture thereof. The buffer may be citric acid and sodium citrate, and/or the isotonic agent may be at least one selected from the group consisting of mannitol, lactose, dextrose and trehalose.

The pH of the composition may range from 4 to 6.

The composition may not include EDTA or a salt thereof.

The composition may be in the form of a sterile injectable preparation.

The pharmaceutically acceptable container may be glass or plastic, for example a vial or an ampoule.

The pharmaceutically acceptable container may be in a volume of 3 to 5 ml.

In another aspect, the present invention relates to a method of improving the storage stability of palonosetron, which comprises a step of introducing a pharmaceutical composition including palonosetron or a pharmaceutically acceptable salt thereof to a pharmaceutically acceptable container, wherein the pharmaceutically acceptable container maintains zinc (Zn) elution amount at 50 ppb or less.

The method of improving the storage stability may maintain the storage stability of palonosetron or a pharmaceutically acceptable salt thereof for 24 months or more.

### [Advantageous Effect]

In accordance with the present invention, the pharmaceutical package including palonosetron and/or a pharmaceutically acceptable salt thereof can be stored for a long time with maintaining pharmaceutically acceptable stability, and can provide a liquid formulation of palonosetron which enables terminal sterilization and has increased stability of palonosetron even without disodium EDTA as a chelating agent.

### [Mode for Invention]

Hereinafter, the present invention will be described in detail.

In one embodiment of the present invention, the pharmaceutical composition comprises at least one selected from the group consisting of palonosetron and its pharmaceutically acceptable salt as an active ingredient.

Palonosetron is a tricyclic 5-HT3 receptor antagonist used as an anti-emetic, and has prophylactic and/or therapeutic effects on nausea and/or vomiting. In particular, palonosetron is useful as an anti-emetic against for vomiting that occurs after surgery, chemotherapy, and radiotherapy. Palonosetron has a chemical name of (*3aS*)-2-[(3S)-1-azabicyclo[2.2.2]oct-3-yl]-2,3,3*a*,4,5,6-hexahydro-1*H*-benz[*de*]isoquinolin-1-one (C₁₉H₂₄N₂O) and a molecular weight of 296.407 g/mol, and has the following Chemical Formula 1.

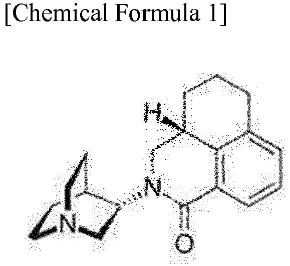

Of them, palonosetron hydrochloride has a chemical name of (3aS)-2-[(S)-1-azabicyclo[2.2.2]oct-3-yl]-2,3,3a,4,5,6-hexahydro-1-oxo-1*H*-benz[*de*]isoquinoline hydrochloride (C₁₉H₂₄N₂O.HCl) and a molecular weight of 332.87 g/mol.

The palonosetron or pharmaceutically acceptable salt thereof is classified as a second-generation 5-HT3 receptor antagonist, and has a half-life of 40 hours or longer. Since the palonosetron or pharmaceutically acceptable salt thereof binds to 5-HT3 receptor for a long time to exhibit the effect for a long time, its inhibitory effect on delayed vomiting is 10 times higher than that of other first-generation 5-HT3 receptor antagonists, thereby being effectively applied to prevention and/or treatment of nausea and/or vomiting caused by chemotherapy such as cytotoxic anticancer agents, etc., radiotherapy, and/or surgery.

However, palonosetron has very poor stability, because it is easily oxidized and degraded by light or oxygen in a liquid medium. Therefore, it is necessary to secure stability of the drug. In particular, oxidation is known to be further accelerated by heavy metals present in the liquid medium. The heavy metals may be intermixed in palonosetron itself, or intermixed from an excipient such as a buffering agent or an isotonic agent which is added during preparation of the liquid formulation. The heavy metals may be also intermixed during preparation processes such as dissolution, filling, packaging, etc.

Therefore, in order to effectively prevent oxidation of the active ingredient palonosetron and/or oxidation accelerated by a trace amount of the heavy metals, it is necessary to remove a trace of the heavy metals or to prevent intermixing of the heavy metals.

The pharmaceutical composition including palonosetron, according to the present invention, may be stably stored for a long period of time even without a chelating agent, for example, it may be stably maintained at room temperature for 2 years or longer. Specifically, the pharmaceutical composition according to the present invention may maintain the content of palonosetron or a pharmaceutically acceptable salt thereof, for example, palonosetron hydrochloride, at 95% by weight to 100% by weight of the initial content thereof, when stored at 25°C for 24 months.

The pharmaceutical composition according to the present invention may include one or more selected from the group consisting of palonosetron and a pharmaceutically acceptable salt thereof as an active ingredient, and a pharmaceutically acceptable aqueous carrier and/or a buffering agent, an isotonic agent, or a mixture thereof.

In an embodiment of the present invention, to effectively obtain the desired effects, a concentration of the active ingredient palonosetron may be 0.01 mg/ml to 0.5 mg/ml, 0.03 mg/ml to 0.2 mg/ml, 0.04 mg/ml to 0.07 mg/ml, or about 0.05 mg/ml.

The pharmaceutically acceptable salt of palonosetron may be one which is pharmaceutically acceptable and possesses the desired pharmacological effect, and examples thereof may include acid addition salts formed with inorganic acids such as hydrochloric acid, bromic acid, sulfuric acid, nitric acid, phosphoric acid, etc.; or organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxybenzoyl)benzoic acid, cinnamic acid, mandelic acid, methanesulfonic acid, ethanesulfonic acid, 1,2,-ethanedisulfonic acid, 2-hydroxyethanesulfonic acid, benzenesulfonic acid, p-chlorobenzenesulfonic acid, 2-naphthalenesulfonic acid, p-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, etc.

According to the present invention, pH of the pharmaceutical composition may be 4 to 6, preferably 4.5 to 5.5, for example, 5. To more stably maintain the pH range of the composition, an organic acid buffering agent such as citric acid and a salt of citric acid may be used. The buffering agent may be an organic acid buffering agent having high buffering capacity. The content of the buffering agent, in the case of citrate buffering agent, may be 10 mM to 100 mM, based on the total pharmaceutical composition. The citrate buffering agent may be used at a concentration of preferably 10 to 40 mM, and more preferably, 15 to 30 mM, based on the total pharmaceutical composition.

The isotonic agent may be one or more selected from the group consisting of mannitol, lactose, dextrose, and trehalose, and preferably, mannitol. In case that the isotonic agent is mannitol, mannitol may be included in an amount of 10 to 80 mg/ml, preferably 20 to 60 mg/ml, and more preferably 40 to 45 mg/ml, based on the total pharmaceutical composition.

The pharmaceutical composition containing palonosetron according to the present invention can be formulated into various forms for oral or parenteral administration and included in a pharmaceutical package. In an embodiment, the pharmaceutical composition may be formulated into various liquid formulations for oral, intravenous, intramuscular, transdermal, intranasal, subcutaneous, or topical administration, and may be formulated into a liquid form as well as a lyophilized form. Generally, the pharmaceutical composition may be formulated into sterile injectable formulations, such as sterile intravenous formulations, by adjusting a final volume with water for injection and performing final sterilization process. In another example, the injectable formulation may be a freeze-dried formulation as well as an injectable solution, but is not limited thereto. The lyophilized formulation of the liquid pharmaceutical composition may be in the form in which the aqueous medium has been removed from the liquid pharmaceutical composition, and have the same or equivalent composition as compared with the above-mentioned palonosetron-containing pharmaceutical composition.

Because both formulations should be formulated in liquid form for intravenous injection, they have the advantage of maintaining stability for a sufficient period of time after they are prepared in liquid form.

According to an embodiment of the present invention, as the pharmaceutically acceptable packaging container is a primary packaging container which is pharmaceutically acceptable, for example, preferably a plastic container from which the elution amount of heavy metals is limited during storage, a glass container surface-treated with silicone, etc., or a glass container from which the elution amount of heavy metals is limited during storage. A rubber stopper, if needed for sealing, may preferably satisfy such requirement.

In an embodiment of the present invention, a pharmaceutically acceptable primary packaging container is characterized in that the amount of eluted zinc (Zn) is maintained at 50 ppb or less. The zinc elution amount may be preferably 30 ppb or less, more preferably 15 ppb or less. In particular, the zinc elution amount may be 13.22 ppb or less, more particularly 0.26 ppb or less.

In addition, the pharmaceutically acceptable container may have calcium (Ca) elution amount of 30 ppb or less, preferably 10 ppb or less, more preferably 3 ppb or less.

In one embodiment of the present invention, the liquid injectable composition of palonosetron except for EDTANa was filled in Wheaton glass vials, sealed and shaded, and the stability test was conducted at 60°C for 6 months, thereby confirming that the liquid injectable composition met the product standard.

Typical element elution patterns of type 1 glass vials commercially available in Korea were found to be varied depending upon manufacturers. Vials containing the eluate treated as described in alkali release test, <Glass containers for pharmaceutical use>, European Pharmacopoeia, were stored for about 3 months and analyzed by ICP-MS. Especially, Zn elution amounts were significantly varied depending on manufacturers. Specifically, while the elution amount from Wheaton's 5 ml vial was only 0.26 ppb, the elution amount from the Huvena's vial was 315.87 ppb, which could be relevant to the result that higher stability was obtained when Wheaton glass vial was used as the primary packaging material in the stability test. In case of Dongsin Tube Glass' vials, the European standard product manufactured by slow processing (according to the manufacturer) showed Zn elution amount of 88 ppb and the Korean standard product manufactured by general processing (according to the manufacturer) showed Zn elution amount of and 157 ppb. It could be inferred from this that vial manufacturing process has relatively large influence on the elution of impurities.

According to one embodiment of the present invention, a glass tubing vial can be used as a pharmaceutically acceptable primary packaging container.

The glass is a transparent borosilicate glass, in particular, a low-expansion borosilicate glass, satisfying ASTM (American Society for Testing and Materials) type 1, class A, USP (US Pharmacopoeia) type 1 Powdered Glass, USP Arsenic, EP (European Pharmacopoeia) type 1 Glass Grains (Test B), and EP Arsenic conditions.

The glass may include SiO₂ ≥ 80%, for example, 80∼85%; Al₂O₃ ≤ 5%, for example, 1∼5%; Na₂O + K₂O ≤ 5%, for example, 1∼5%; CaO + MgO < 0.2%, for example, more than 0% and less than 0.2%; B₂O₃ ≥ 10%, for example, 10∼15%; Fe₂O₃ < 0.1%, for example, more than 0% and less than 0.1%, and may not include BaO, ZnO, MnO₂, TiO₂, SO₃. For example, the glass may include SiO₂ 81%, Al₂O₃ 2%, Na₂O + K₂O 4%, CaO + MgO < 0.2%, B₂O₃ 13%, Fe₂O₃ < 0.1%, and may not include BaO, ZnO, MnO₂, TiO₂, SO₃.

The glass may have a strain point of less than 510°C, preferably more than 500°C and less than 510°C, and most preferably 505°C; an annealing point of 550∼570°C, preferably 555∼565°C, and most preferably 560°C; a softening point of 800∼900°C, preferably 800∼850°C, and most preferably 820°C; a linear coefficient of expansion (0-300°C) of 30 x10⁻⁷ ∼40x10⁻⁷, preferably 30 x10⁻⁷ ∼ 35x10⁻⁷, and most preferably 33x10⁻⁷; and a density of 2.2∼2.3 g/cm³, preferably 2.2∼2.25 g/cm³, and most preferably 2.22 g/cm³.

The pharmaceutical package according to the present invention may be stored and/or distributed before use, in which the pharmaceutical composition comprising palonosetron and other ingredients is filled and sealed, for example in the form of a vial or an ampoule. The storage may be performed under aseptic (sterile) environments (e.g., a clean room). To prevent a photochemical reaction by direct sunlight and degradation and/or denaturation of active ingredients caused thereby in the vial or ampoule, a brown-colored container may be used or the vial or ampoule may be stored in the dark. For example, in case that the pharmaceutical composition is prepared in the form of a sterile injectable formulation, it may be filled and sealed in a vial or ampoule made of glass or plastic, and then stored and/or distributed under aseptic (sterile) environments (e.g., a clean room). Further, to prevent a photochemical reaction by direct sunlight, a brown-colored container may be used or the composition may be stored in the dark. The pharmaceutical package enables terminal sterilization.

### [Detailed Description of the Embodiments]

The present invention will be described in more detail with reference to the following Examples. However, these Examples are for illustrative purposes only, and the invention is not intended to be limited by these Examples.

### Example 1: Stability Test for aqueous solution of palonosetron hydrochloride with various primary packaging materials

Aqueous solution of palonosetron hydrochloride without EDTA Na was prepared according to the composition of the following Table 1 and stored at 60°C. According to the analysis method of related substances of palonosetron hydrochloride, active pharmaceutical ingredient (Emcure), the related substances were analyzed and the total amount of related substances except for diastereomer was obtained.

### (1) Sample

**[Table 1] Composition in 1 ml of aqueous solution of palonosetron hydrochloride**

| Components | Amount |
|---|---|
| Palonosetron hydrochloride | 0.056 mg |
| D-mannitol | 41.5 mg |
| Sodium citrate | 3.7 mg |
| Citric acid monohydrate | 1.56 mg |
| Water for injection | 1 ml |

### (2) Storage Conditions and Period

The samples were sealed using an aluminum seal and a rubber stopper, and allowed to stand at 60°C for 2 weeks under light shielded condition.

### (3) Analysis Method

Analysis was performed by HPLC according to the following method.
a) Preparation of Buffer
3.1 g of sodium dihydrogen orthophosphate and 2.5 mL of triethylamine were accurately weighed and added to a flask with a volume of 1000 mL, and dissolved in purified water to the marked line. pH of this solution was adjusted to 7.0 ± 0.05 using phosphate. Before pH measurement, a pH meter was corrected by standard buffers of 1) 6.00 and 2) 8.00. Filtration was performed using a filter paper of 0.45 µm, followed by degassing.
b) Preparation of Mobile Phase A
The prepared buffer was used as Mobile Phase A.
c) Preparation of Mobile Phase B
Acetonitrile was used as Mobile Phase B.
* Caution: mobile phase was used within 16 hours after preparation.
d) Diluent
A solution was prepared by homogenously mixing the buffer and acetonitrile at a volume ratio of 50:50.
e) Chromatography Conditions
Column: 150 mm×4.6 mm, 5 µm, Intersil C8 or equivalent thereto
Column flow rate: 1.0 mL/min
Detector: UV wavelength: 210 nm
Injection volume: 20 µl
Column temperature: 40°C
Analysis time: 45 minutes
Gradient elution program: according to the following Table 2

**[Table 2]**

| Time (min) | Mobile phase A (%) | Mobile phase B(%) |
|---|---|---|
| 0.01 | 80 | 20 |
| 2.0 | 80 | 20 |
| 25.0 | 65 | 35 |
| 35.0 | 65 | 35 |
| 37.0 | 80 | 20 |
| 45.0 | 80 | 20 |

f) A total amount of unidentified related impurities (%) = A sum of peak areas of respective unidentified related impurities of test sample / a peak area of palonosetron of test sample × 100

### (4) Results of stability test

As shown in the following Table 3, solutions excluding EDTANa were filled and sealed in clear glass vials provided by manufacturers W and Y and plastic vials provided by Daikyo as primary packaging materials.

After being stored at 60°C for 2 weeks, the total amount of the unidentified related impurities was examined. When the packaging material of the manufacturer W was used, the total amount of the unidentified related impurities was 0.1∼0.3%, suggesting that this packaging material is more stable than other packaging materials.

### [Table 3]

Total amount of unidentified related impurities (%) in the composition of the present invention according to packaging materials

| Manufacturer /sample | W* | H** | Daikyo(CD)*** |
|---|---|---|---|
| Immediately after preparation | 0.1% | N.D. | 0.1% |
| After storage at 60°C/2 weeks | 0.3% | 3.2% | 1.6% |

| | | | |
|---|---|---|---|
| (W: Wheaton, H: Huvena) | | | |

### Example 2: Test for typical element elution from primary packaging material (glass vial)

As the result of Example 1, the glass vial of Wheaton was selected as primary packaging material. As steam sterilization is included in the product manufacturing process, the primary packaging material suitable for steam sterilization was selected.

### (1) Sample

The glass vials selected as the primary packaging materials were compared with commercial products currently widely used for general liquid injections.

**[Table 4] Domestically supplied liquid injection vials**

| Sample name | Manufacturer | Glass type | volume | Processing |
|---|---|---|---|---|
| W3 | W | Type 1 glass | 3ml | General processing |
| W5 | W | Type 1 glass | 5ml | General processing |
| Y10 | Y | Type 1 glass | 10ml | General processing |
| D3EP | D | Type 1 glass | 3ml | Slow processing |
| D5EP | D | Type 1 glass | 5ml | Slow processing |
| D3KP | D | Type 1 glass | 3ml | General processing |
| D5KP | D | Type 1 glass | 5ml | General processing |
| H3 | H | Type 1 glass | 3ml | General processing |
| H5 | H | Type 1 glass | 5ml | General processing |

| | | | | |
|---|---|---|---|---|
| (W: Wheaton, Y: Yeonhap Glass, D: Dongsin Tube Glass Ind. Co., Ltd, H: Huvena) | | | | |

### (2) Test method

The sample was treated as disclosed in alkali release test of European Pharmacopoeia Ver.5.0, 3.2.1 <Glass containers for pharmaceutical use>, and then the eluted heavy metals were analyzed by ICP-MS and ICP-AES.

### (3) Determination of Filing volume

i) 17 vials of 3ml and 10 vials of 5ml were selected randomly.
ii) The impurities were removed and an empty vial was weighed accurately to one decimal place.
iii) The glass vials were placed on a flat surface and filled with ultrapure water.
iv) The glass vials filled with ultrapure water were weighed to two decimal places.
v) The average weight of each glass vial was calculated and multiplied by 0.9. This volume was represented to one decimal place, which represented the filling volume of the lot.

### (4) Surface test

i) Dust and foreign matter were removed from the glass vial, washed at least twice with purified water, and filled with purified water.
ii) The glass vial was rinsed with purified water just before testing, finally rinsed with ultra-pure water and drained.
iii) The cleaning process was performed within 20 to 25 minutes.
iv) The glass vial was thoroughly washed with ultrapure water and dried in a dry oven at about 50 °C.
v) The glass vial was filled with ultrapure water up to the filling volume and capped with rubber stopper pretreated with ultrapure water (3 ml vial: W224100-081, 5 ml vial: W224100-181).
vi) The glass vial with rubber stopper was autoclaved at 121 ± 1 °C for 60±1 min.
vii) The extract solution was taken from the autoclave, and cooled at room temperature uncontaminated.
viii) The constant amount of the extract solution was analyzed.

### (5) Analysis method

### [1] ICP-AES

The treated samples were analyzed within a week, and the heavy metals to be analyzed were Fe, Cu, Mn, Pb and Zn.

Analysis was performed using ICP/AES (Inductively coupled plasma / Atomic Emission Spectrometer, model name: OPTIMA 5300DV, Perkin Elmer) under the following conditions.

### <Instrumental conditions>

RF Power: 1300 watts
Nebulizer Ar Flow: 0.65 L/min
Plasma Ar Flow: 15 L/min
Pump Flow rate: 1.5 mL/min
View Dist.: 15 mm
Read Delay time: 30 sec

### Sample Pretreatment

About 100 mg of the sample was accurately weighed using a microwave digestion system (model: Multiwave 3000, manufacturer: Anton Paar), and subjected to complete acid digestion for about 2 hours using an acid mixture of nitric acid (5 mL) and hydrogen peroxide (1 mL). Then, the sample was subjected to filtration, followed by finally massing up to 50 ml. All pretreatment procedures were performed using a Teflon container (metal free), not a glass container, in order to avoid contamination.

### Preparation of Standard Solution and Measurement

An inorganic element standard for ICP (Merck, USA) of 1,000 ppm was used as a standard solution, and diluted with an acid mixture (sample blank) which was pretreated in the same manner as the sample. Calibration curves of the corresponding concentrations were constructed, and then ICP test was performed.

### [2] ICP-MS

The vials including treated samples were left for 3 months at room temperature and analyzed, and the heavy metals to be analyzed were Fe, Cu, Mn, Pb and Zn.

### Analysis results of heavy metals

When being analyzed by ICP-AES immediately after eluting heavy metals from the vials, most of the ions including heavy metals were not detected. This may be because the detection limit of ICP-AES is higher than that of ICP-MS or the heavy metals had not been eluted due to no storage period.

The vial containing the elute solution was stored for about 3 months and analyzed by ICP-MS. As shown in the following table, Zn amount varied significantly. The elution amount from Wheaton's 3 ml vial was only 0.26 ppb, but the elution amount from the Huvena vial was 315.87 ppb, which was considered to be related to the result that Wheaton's glass vial as the primary packaging material showed a higher stability than other vials in the stability test. In the case of the vials of Dongsin Tube Glass Ind. Co., Ltd, the Zn elution amounts of the EP standard product manufactured by slow processing method (according to the manufacturer) and the KP standard product manufactured by general processing method (according to the manufacturer) were 88 ppb and 157 ppb, respectively. It could be considered that the vial manufacturing process has relatively large influence on the elution of impurities.

**[Table 5] ICP-MS Test result**

| Element | Measured content(ppb) | | | | | |
|---|---|---|---|---|---|---|
| | Wheaton -3ml | Wheaton -5ml | Huvena | Yeonhap glass | Dongsin Tube Glass Ind. Co., Ltd for EP | Dongsin Tube Glass Ind. Co., Ltd for KP |
| Cd | <0.01 | <0.01 | <0.01 | <0.01 | <0.01 | <0.01 |
| Co | <0.02 | <0.02 | <0.02 | <0.02 | <0.02 | <0.02 |
| Cr | <0.1 | <0.1 | <0.1 | <0.1 | <0.1 | <0.1 |
| Cu | <0.1 | <0.1 | <0.1 | <0.1 | <0.1 | <0.1 |
| Fe | <2 | <2 | <2 | <2 | <2 | <2 |
| Hg | <0.05 | <0.05 | <0.05 | <0.05 | <0.05 | <0.05 |
| As | <0.06 | <0.06 | <0.06 | <0.06 | <0.06 | <0.06 |
| Mn | <0.05 | <0.05 | <0.05 | <0.05 | <0.05 | <0.05 |
| Ni | <0.05 | <0.05 | <0.05 | <0.05 | <0.05 | <0.05 |
| Pb | <0.01 | <0.01 | <0.01 | <0.01 | <0.01 | <0.01 |
| **Zn** | **0.26** | **13.22** | **315.87** | **52.89** | **88.35** | **156.76** |
| Al | <0.1 | <0.1 | <0.1 | <0.1 | <0.1 | <0.1 |
| Ag | <0.1 | <0.1 | <0.1 | <0.1 | <0.1 | <0.1 |
| Au | <0.5 | <0.5 | <0.5 | <0.5 | <0.5 | <0.5 |
| Ba | <0.5 | <0.5 | <0.5 | <0.5 | <0.5 | <0.5 |
| Ca | <3 | 28.86 | 48.67 | 28.57 | 52.81 | 50.50 |
| K | <3 | <3 | <3 | 74.23 | <3 | <3 |
| Li | <0.2 | <0.2 | <0.2 | <0.2 | <0.2 | <0.2 |
| Mg | <2.5 | <2.5 | <2.5 | <2.5 | <2.5 | <2.5 |
| Mo | <0.01 | <0.01 | <0.01 | <0.01 | <0.01 | <0.01 |
| Pd | <0.1 | <0.1 | <0.1 | <0.1 | <0.1 | <0.1 |
| Pt | <0.1 | <0.1 | <0.1 | <0.1 | <0.1 | <0.1 |
| Sn | <0.01 | <0.01 | <0.01 | <0.01 | <0.01 | <0.01 |
| Ti | <0.1 | <0.1 | <0.1 | <0.1 | <0.1 | <0.1 |
| Tl | <0.01 | <0.01 | <0.01 | <0.01 | <0.01 | <0.01 |
| V | <0.01 | <0.01 | <0.01 | <0.01 | <0.01 | <0.01 |
| W | <0.05 | <0.05 | <0.05 | <0.05 | <0.05 | <0.05 |
| Zr | <0.05 | <0.05 | <0.05 | <0.05 | <0.05 | <0.05 |
| **Na** | **980.96** | **1015.73** | **178.41** | **422.01** | **558.10** | **376.06** |

### Example 3: Commercial scale production and test of aqueous solution of palonosetron hydrochloride

The raw materials were weighed as shown in Table 6 and introduced to the liquid injection preparation tank (batch size 400 ml). D-mannitol, sodium citrate, and citric acid monohydrate were mixed and dissolved, and then palonosetron hydrochloride was mixed and dissolved therein. The pH of the resulting mixture was adjusted by adding a pH adjusting agent (hydrochloric acid and/or sodium hydroxide), if necessary, and aseptically filtered and aseptically filled in Wheaton's 3 ml vial. At this time, the filling was manually performed in a clean bench at an amount of 1.5 ml per vial.

**[Table 6] Introduced amount of raw materials**

| Components | Standard amount | Introduced amount |
|---|---|---|
| Palonosetron hydrochloride | 22.4 mg | 22.4 mg |
| D-mannitol | 16.6 g | 16.6 g |
| Sodium citrate | 1.480 g | 1.480 g |
| Citric acid monohydrate | 0.624 g | 0.624 g |
| Water for injection | 389.5 g | 400 g |

The test results of aqueous solution of palonosetron hydrochloride are shown in Table 7.

**[Table 7] Test results of aqueous solution of palonosetron hydrochloride**

| Test items | Standard | Test results |
|---|---|---|
| Description | Colorless transparent injectable liquid | Colorless transparent injectable liquid |
| Identification | 1)Identical peak retention time | 1) Identical peak retention time |
| | 2)Identical absorption spectrum | 2)Identical absorption spectrum |
| pH | 4.5∼5.5 | 4.97 |
| Sterility | Negative | Negative |
| Bacterial Endotoxins | 60EU/mg or lower | 10EU/mg or lower |
| Insoluble Particulate Matter | 10*µ*m or higher: ≤6000 | 10*µ*m or higher: ≤18 |
| | 25*µ*m or higher: ≤600 | 25*µ*m or higher: 0 |
| Foreign insoluble Matter | Not detected | Not detected |
| Related Substance | Related Substance A: ≤1.0% | Related Substance A: N.D. |
| | Diastereomer: ≤1.0% | Diastereomer: N.D. |
| | Other individuals: ≤1.0% | Other individuals: N.D. |
| | Total: ≤2.0% | Total: N.D. |
| Content | 90.0∼110.0% | 102.3% |

As shown in Table 7, the aqueous solution of palonosetron hydrochloride was found to satisfy all test items.

While the present invention has been particularly shown and described with reference to exemplary embodiments thereof, it is to be understood that the invention is not limited to the disclosed exemplary embodiments. Accordingly, the substantial scope of the present invention will be defined by the appended claims and their equivalents.

## Claims

1. A pharmaceutical package of palonosetron, comprising a pharmaceutical composition comprising a palonosetron or a pharmaceutically acceptable salt thereof, and a pharmaceutically acceptable container, **characterized in that** the pharmaceutically acceptable container maintains the elution amount of zinc (Zn) at 50 ppb (parts per billion) or lower.

2. The pharmaceutical package of palonosetron of claim 1, **characterized in that** the pharmaceutically acceptable container maintains the elution amount of calcium (Ca) at 30 ppb (parts per billion) or lower.

3. The pharmaceutical package of palonosetron of claim 1 or claim 2, **characterized in that** the pharmaceutical composition further comprises an aqueous carrier.

4. The pharmaceutical package of palonosetron of claim 1 or claim 2, **characterized in that** the pharmaceutical composition comprises palonosetron or a pharmaceutically acceptable salt thereof at a concentration of 0.01 to 0.5 mg/ml as palonosetron.

5. The pharmaceutical package of palonosetron of claim 1 or claim 2, **characterized in that** the pharmaceutical composition further comprises a buffering agent, an isotonic agent, or a mixture thereof,

6. The pharmaceutical package of palonosetron of claim 5, **characterized in that** the buffering agent is citric acid and sodium citrate, or the isotonic agent is one or more selected from the group consisting of mannitol, lactose, dextrose, and trehalose.

7. The pharmaceutical package of palonosetron of claim 1 or claim 2, **characterized in that** pH of the composition ranges from 4 to 6.

8. The pharmaceutical package of palonosetron of claim 1 or claim 2, **characterized in that** the pharmaceutical composition is free of EDTA or a salt thereof.

9. The pharmaceutical package of palonosetron of claim 1 or claim 2, **characterized in that** the pharmaceutical composition is in the form of sterile injectable formulation.

10. The pharmaceutical package of palonosetron of claim 1 or claim 2, **characterized in that** the pharmaceutically acceptable container is a glass or plastic container.

11. The pharmaceutical package of claim 10, **characterized in that** the pharmaceutically acceptable container is a vial or an ampule.

12. A method of increasing storage stability of palonosetron, comprising introducing a pharmaceutical composition comprising a palonosetron or a pharmaceutically acceptable salt thereof, into a pharmaceutically acceptable container, **characterized in that** the pharmaceutically acceptable container maintains the elution amount of zinc (Zn) at 50 ppb (parts per billion) or lower.

13. The method of increasing storage stability of palonosetron of Claim 12, **characterized in that** the storage stability of palonosetron or a pharmaceutically acceptable salt thereof is maintained for 24 month or longer.
